# EUROPEAN PATENT APPLICATION

(11) **EP 3 960 186 A2**
(43) Date of publication of application: **02.03.2022**
(21) Application number: 21188687.4
(22) Date of filing: 01.10.2015
(51) Int. Cl.: A61K 35/00, A61K 31/00, A61K 33/00, A61Q 1/00, A61K 8/00

(54) **PROMOTING BONE HEALTH AND REPAIR AND TREATING DISORDERS RELATED TO COLLAGEN AND PERTINENT PROTEINS BY USING SHILAJIT**

(30) Priority: 02.10.2014 US 201462059072 P
(62) Divisional of application: 15847241.5
(71) Applicant: Natreon, Inc., New Brunswick, NJ 08901 (US)
(72) Inventor: SEN, Chandan K., Columbus, OH, 43220 (US)
(74) Representative: FRKelly

(57) **Abstract**

A method of using Shilajit or its individual components, or a combination of two or more of these components, to induce the body of a mammal, including that of a human, to synthesize new collagen and the related extracellular matrix proteins, thus promoting the health of all of the tissues and organs containing collagen and the related extracellular matrix proteins, including skin, connective tissue, muscle, cartilage, bone, and teeth and improve muscle building and regeneration, and/or treat collagen-related disorders is presented.

## Description

### TECHNICAL FIELD

The present invention relates to promoting collagen synthesis and thus improving muscle building and repair and the health of and/or treating diseases of skin, cartilage, connective tissues, muscle, vascular tissues, bones, and teeth in the body of a mammal, including a human, through the use of: Shilajit; its individual chemical constituents, including 3-hydroxy-dibenzo-α-pyrone, 3,8-dihydroxy-dibenzo-α-pyrone, dibenzo-α-pyrone chromoproteins, humic acid, fulvic acid, and more than forty (40) minerals; or combinations thereof.

### BACKGROUND

Collagen is the body's major structural protein composed of three protein chains wound together in a tight triple helix. This unique structure gives collagen a greater tensile strength than steel. Approximately thirty-three (33) percent of the protein in the body is collagen. This protein supports tissues and organs and connects these structures to bones. In fact, bones are also composed of collagen combined with certain elements such as calcium and phosphorus. Collagen plays a key role in providing the structural scaffolding surround cells that helps to support cell shape and differentiation. The mesh-like collagen network binds cells together and provides the supportive framework or environment in which cells develop and function, and tissues and bones heal.

Collagen, in the form of elongated fibrils, is mostly found in fibrous tissues such as tendons, ligaments, and skin. Collagen is also abundant in corneas, cartilage, bones, blood vessels, the gut, intervertebral discs, and the dentin in teeth. In muscle tissue, collagen serves as a major component of the endomysium. Collagen constitutes one to two percent of muscle tissue, and accounts for six (6) percent of the weight of strong, tendinous muscles. The fibroblast is the most common cell that creates collagen.

### Types of Collagen:

Collagen occurs in many places throughout the body. Over ninety (90) percent of the collagen in the body is type I. So far, twenty-eight (28) types of collagen have been identified and described. The five (5) most common types are:
- Collagen I: skin, tendon, vascular ligature, organs, bone (main component of the organic part of bone);
- Collagen II: cartilage (main component of cartilage);
- Collagen III: reticulate (main component of reticular fibers), commonly found alongside Collagen I;
- Collagen IV: forms basal lamina, the epithelium-secreted layer of the basement membrane; and
- Collagen V: cell surfaces, hair, and placenta.

### Role of Collagen in the Body:

Collagen is important to health because it plays a key role in maintaining the health of skin, connective tissues, tendons, bones, and cartilage as detailed below:

Skin health: Collagen plays an important role in skin health. Collagen I and Collagen III are formed in human skin in a higher proportion relative to other types of collagen and are maintained in a fixed proportion relative to one another in normal skin tissue. Collagen I constitutes about seventy (70) percent of collagen in the skin, with Collagen III constituting about ten (10) percent of collagen in the skin and Collagens IV, V, VI, and VII each constituting trace amounts of collagen in the skin. Collagen maintains firmness and elasticity of the skin. Collagen, in the form of collagen hydrolysate, keeps skin hydrated. Decreases in the amount of collagen in the body with age result in sag, lines, wrinkles, lack of tension and elasticity, and delay in wound healing processes.

Wound healing: Collagen is a key protein in connective tissue and plays an important role in wound healing by repair and formation of scar. Age-related delay in wound healing is caused by impaired synthesis and increased degradation of collagen.

Bone: About 95% of the organic part of the bone is made of collagen, mainly Collagen I. The combination of hard minerals and flexible collagen makes bone harder than cartilage without being too brittle. Combination of collagen mesh and water forms a strong and slippery pad in the joint that cushions the ends of the bones in the joint during muscle movement.

Cartilage, tendon, ligaments: Collagen, in the form of elongated fibrils, is predominantly found in fibrous tissues such as tendons and ligaments. It is a flexible and stretchy protein that is used by the body to support tissues and thus it plays a vital role in the maintenance of the cartilage, tendons, and ligaments. Normal tendon consists of soft and fibrous connective tissue that is composed of densely packed collagen fiber bundles and that is surrounded by a tendon sheath. Collagen II is the major component in cartilage.

### Muscles: In muscle tissue, collagen serves as a major component of the endomysium.

Dental tissue: The organic part of dentin and pulp consist of collagen, mainly Collagen I, with small amounts of Collagens III and V. The predominant collagen found in cementum is Collagen I, and in periodontal ligament, are Collagens I, III, and XII.

Basement membrane: The epithelial basement membrane is composed of Collagens IV and VII.

### Collagen-related Disorders:

Collagen-related disorders most commonly arise from genetic defects or nutritional deficiencies that affect the biosynthesis, assembly, post-translational modification, secretion, or other processes involved in normal collagen production. Various collagen-related disorders are described below:

Osteogenses imperfecta is a dominant autosomal disorder caused by a mutation in Collagen I. Osteogenses imperfecta results in weak bones and irregular connective tissue. Some cases can be mild while severe cases can be lethal. Mild cases are characterized by lowered levels of Collagen I while severe cases are characterized by structural defects in collagen.

Chondrodysplasias is a skeletal disorder believed to be caused by a mutation in Collagen II and the subject of continuing research efforts.

Ehlers-Danlos Syndrome leads to deformities in connective tissues. There are ten different types of this disorder that are known, some of which are characterized by the rupturing of arteries and are thus lethal. Each type of the Ehlers-Danlos Syndrome is caused by a different mutation; for example, type four (4) of this syndrome is caused by a mutation in Collagen III.

Alport Syndrome can be passed on genetically, usually as an X-linked dominant gene, but also as both an autosomal dominant and autosomal recessive gene. Individuals suffering from Alport Syndrome experience kidney and eye problems, and childhood or adolescent loss of hearing.

Osteoporosis is experienced with age rather than inherited genetically, and is associated with reduced levels of collagen in the skin and bones. Growth hormone injections are being researched as a possible treatment for osteoporosis in order to counteract any loss of collagen.

Knobloch syndrome is caused by a mutation in the Collagen XVIII gene. Patients suffering from Knobloch syndrome present with protrusion of the brain tissue and degeneration of the retina. Individuals who have one or more family members suffering from Knobloch syndrome are at an increased risk of developing it themselves, as there is a hereditary predisposition.

### Degradation and Decreased Production of Collagen:

With age, collagen degrades, and there is a decrease in the production of collagen. As a result, fine lines and wrinkles appear in the skin. Skin also loses its elasticity and sags. Collagen can be preserved by reducing degradation of existing collagen and increasing the production of new collagen. Degradation of collagen can be reduced by: (a) protecting the skin from UVA and UVB rays; (b) avoiding excessive exposure to sunlight; (c) having a diet including antioxidants to fight free radicals; (d) ingesting Vitamin C, which accelerates production of new collagen; (e) supplementing with collagen-stimulating peptides; and (f) increasing the intrinsic ability of the body to produce new collagen.

A brief description of several proteins in muscle and connective tissue is warranted in order to understand the significance of the present invention.

Tenascin XB is a member of the tenascin family, tenascin X (TN-X), also known as hexabrachion-like protein. Tenascin XB is a glycoprotein that is expressed in connective tissues including skin, joints, and muscles. In humans, tenascin XB is encoded by the TNXB gene.

Decorin is a component of connective tissue, binds to Collagen I fibrils, plays a role in matrix assembly, and is encoded by the DCN gene. Decorin appears to influence fibrillogenesis, and also interacts with fibronectin, thrombospondin, the complement component C1q, epidermal growth factor receptor ("EGFR"), and transforming growth factor-beta ("TGF-beta"). Decorin has been shown to either enhance or inhibit the activity of TGF-beta 1. The primary function of decorin involves regulation during the cell cycle. It is involved in the regulation of autophagy of endothelial cell and inhibits angiogenesis. This process is mediated by a high-affiinity interaction with vascular endothelial growth factor receptor ("VEGFR2") which leads to increased levels of tumor suppressor gene, called PEG 3. Decorin has recently been established as a myokine. In this role, decorin promotes muscle hypertrophy by binding with myostatin.

Myoferlin is a protein in humans that is encoded by the MYOF gene. Skeletal muscle is a multinucleated syncytium that develops and is maintained by the fusion of myoblasts to the syncytium. Myoferlin is a membrane-anchored, multiple C2 domain-containing protein that is highly expressed in myoblasts and is required for efficient myoblast fusion to myotubes. Thus, myoferlin plays an important role in muscle building and regeneration.

As previously described, there are twenty-eight (28) types of collagen. Collagens I, II, and III are the most predominant types. The COL1A1 gene produces a component of Collagen I, called the pro-alpha1 (I) chain. This chain combines with another pro-alpha1 (I) chain and also with a pro-alpha2 (I) chain (produced by the COL1A2 gene) to make a molecule of type I procollagen. These triple-stranded, rope-like pro-collagen molecules are processed by enzymes outside the cell. Once these molecules are processed, they arrange themselves into long, thin fibrils that cross-link to one another in the spaces around cells. The cross-links result in the formation of very strong, mature Collagen I fibers.

The COL1A2 gene encodes one of the chains for Collagen I, the fibrillar collagen found in most connective tissues. Mutations in this gene are associated with osteogenesis imperfecta, Ehlers-Danlos syndrome, idiopathic osteoporosis, and atypical Marfan syndrome. Symptoms associated with mutations in this gene, however, tend to be less severe than mutations in the gene for alpha-1 Collagen I because alpha-2 is less abundant.

The COL2A1 gene is a human gene that provides instructions for the production of the pro-alpha1 (II) chain of Collagen II. This gene encodes the alpha-1 chain of Collagen II, a fibrillar collagen found in cartilage and the vitreous humor of the eye. Mutations in this gene are associated with achondrogenesis, chondrodysplasia, early onset familial osteoarthritis, SED congenital, Langer-Saldino achondrogenesis, Kniest dysplasia, Stickler syndrome type I, and spondyloepimetaphyseal dysplasia Strudwick type. In addition, defects in processing chondrocalcin, a calcium-binding protein that is the C-propeptide of Collagen II, are also associated with chondrodysplasia. There are two transcripts identified for the COL2A1 gene. Collagen II, which adds structure and strength to connective tissues, is found primarily in cartilage, the jelly-like substance that fills the eyeball (the vitreous), the inner ear, and the center portion of the discs between the vertebrae in the spine (nucleus pulposus). Three pro-alpha1 (II) chains twist together to form a triple-stranded, ropelike procollagen molecule. These procollagen molecules must be processed by enzymes in the cell. Once these molecules are processed, they leave the cell and arrange themselves into long, thin fibrils that cross-link to one another in the spaces around cells. The cross-linkages result in the formation of very strong, mature Collagen II fibers.

Collagen III alpha-1 is encoded by the COL3A1 gene. It is a fibrillar collagen that is found in extensible connective tissues such as skin, lung, and the vascular system, frequently in association with Collagen I.

The COL5A2 gene encodes an alpha chain for one of the low abundance fibrillar collagens. Fibrillar collagen molecules are trimers that can be composed of one or more types of alpha chains. Collagen V is found in tissues containing Collagen I and appears to regulate the assembly of heterotypic fibers composed of both Collagens I and V. This gene product is closely related to Collagen XI and it is possible that the collagen chains of Collagens V and XI constitute a single collagen type with tissue-specific chain combinations.

The COL6A3 gene encodes the alpha 3 chain, one of the three alpha chains of Collagen VI, a beaded filament collagen found in most connective tissues. The alpha 3 chain of Collagen VI is much larger than the alpha 1 and 2 chains. This difference in size is largely due to an increase in the number of subdomains, similar to von Willebrand Factor type A domains, found in the amino terminal globular domain of all the alpha chains. These domains have been shown to bind extracellular matrix ("ECM") proteins, an interaction that explains the importance of this collagen in organizing matrix components.

The COL14A1 gene encodes the alpha chain of Collagen XIV, a member of the fibril-associated collagens with interrupted triple helices ("FACIT") collagen family. Collagen XIV interacts with the fibril surface and is involved in the regulation of fibrillogenesis.

Elastin is a protein in connective tissue that is elastic and allows many tissues in the body to resume their shapes after stretching or contracting. Elastin helps skin to return to its original position when it is poked or pinched. Elastin is also an important load-bearing tissue in the bodies of vertebrates and is used in places where mechanical energy must be stored. In humans, elastin is encoded by the ELN gene.

Fibrillin is a glycoprotein, which is essential for the formation of elastic fibers found in connective tissue. Fibrillin is secreted into the ECM by fibroblasts and becomes incorporated into the insoluble microfibrils, which appear to provide a scaffold for deposition of elastin. It is encoded by the gene FBN1.

Fibronectin is a high-molecular weight glycoprotein of the ECM that binds to membrane-spinning receptor proteins called integrins. Similarly to integrins, fibronectin binds ECM components such as collagen, fibrin, and heparin sulfate proteoglycans (e.g. syndecans). It plays a major role in cell adhesion, growth, migration, and differentiation, and is important for processes such as wound healing and embryonic development.

Mutations in the above-referenced genes are believed to be responsible for one or more Collagen-Related Disorders.

The ECM is essential for the development, maintenance, and regeneration of skeletal muscles. C.A. Buck & A.F. Horwitz, Cell surface receptors for extracellular matrix molecules, 3 ANNU. REV. CELL BIOL. 179 (1987); P.P. Purslow, The structure and functional significance of variations in the connective tissue within muscle, 133 COMP. BIOCHEM. PHYSIOL. A MOL. INTEGR. PHYSIOL. 947 (2002). The ECM is mainly composed of glycoproteins, collagen, and proteoglycans. The ECM is also involved in the macrostructure of muscle, arranging fibers into bundles, bundles into fascicles, and integrating muscle structure with aponeurosis and tendon. Additionally, the ECM is thought to play a vital role in mechano-transduction, transmitting force laterally from the fiber to the tendon and vice versa. G.M. Fomovsky et al., Contribution of extracellular matrix to the mechanical properties of the heart, 48 J. MOL. CELL CARDIOL. 490 (2010); P.P. Purslow & J.A. Trotter, The morphology and mechanical properties of endomysium in series-fibred muscles: variations with muscle length, 15 J. MUSCLE RES. CELL MOTIL. 299 (1994); S.F. Street, Lateral transmission of tension in frog myofibers: a myofibrillar network and transverse cytoskeletal connections are possible transmitters, 114 J. CELL PHYSIOL. 346 (1983). The mechanical strength and elasticity of the ECM are critical to its functional performance—it must be strong enough to sustain the loads of contraction, yet elastic enough to prevent tearing under externally applied strains. P.P. Purslow, *supra,* 133 COMP. BIOCHEM. PHYSIOL. A MOL. INTEGR. PHYSIOL. at 947. These properties change both with age and disease, where chronic alterations to the ECM appear to impair muscle function. R.L. Lieber et al., Inferior mechanical properties of spastic muscle bundles due to hypertrophic but compromised extracellular matrix material, 28 MUSCLE NERVE 464 (2003); L. Zhou & H. Lu, Targeting fibrosis in Duchenne muscular dystrophy, 69 J. NEUROPATHOL. EXP. NEUROL. 771 (2010). Skeletal muscle shows an enormous plasticity to regulate its functional, structural, and metabolic properties in order to adapt to varying physiological demands. McGee and colleagues detected HDAC-specific inhibition patterns and changes in histone acetylation after a single bout of exercise in human skeletal muscle. S.L. McGee et al., Exercise-induced histone modifications in human skeletal muscle, 587 J. PHYSIOL. 5951 (2009).

Physical exercise essentially contributes to a healthy lifestyle and leads to risk reduction, a better prognosis, and a decrease in the medical treatment-specific side effects of several common diseases, including cancer as well as cardiovascular, metabolic, and neurodegenerative disorders. I.M. Lee et al., Effect of physical inactivity on major non-communicable diseases worldwide: an analysis of burden of disease and life expectancy, 380 LANCET 219 (2012); D. Schmid & M.F. Leitzmann, Association between physical activity and mortality among breast cancer and colorectal cancer survivors: a systematic review and meta-analysis, 25 ANN ONCOL. 1293 (2014). For example, short-term exercise training partially reverses the progression of metabolic diseases, whereas lifestyle interventions incorporating increased physical activity remain the primary preventive approach for metabolic diseases. In fact, regular exercise combined with dietary intervention is more successful than pharmacological intervention in the treatment of Type II Diabetes Mellitus ("T2DM") and sarcopenia. W.C. Knowler et al., Reduction in the incidence of type 2 diabetes with lifestyle intervention or metformin, 346 N. ENG. J. MED. 393 (2002); S.E. Borst, Interventions for sarcopenia and muscle weakness in older people, 33 AGE AGEING 548 (2004). While the benefits of adopting regular exercise are long known, molecular biologists have recently uncovered networks of signaling pathways and regulatory molecules that coordinate adaptive responses to exercise. Schmid & Leitzmann, *supra* at 1293; O. Mathieu-Costello & R.T. Hepple, Muscle structural capacity for oxygen flux from capillary to fiber mitochondria, 30 EXERC. SPORT SCI. REV. 80 (2002). Adaptation induced by exercise training results in the expansion of skeletal muscle vascularity and is recognized to be an important means of increasing the conductance of oxygen within the exercising muscle. Schmid & Leitzmann, *supra* at 1293; Mathieu-Costello & Hepple, *supra* at 80. Exercise is known to directly impact the ECM in different tissues. Exercise has been reported to alter the collagen pattern in the heart of exercising rats. H.B. Kwak, Aging, exercise, and extracellular matrix in the heart, 9 J. EXERC. REHABIL. 338 (2013).

There are a variety of collagen supplements available in the market, for oral ingestion as well as topical application, to improve the elasticity and firmness of the aging skin and for improvement of joint health. However, these supplements may be of dubious efficacy, because collagen, being a protein, will be digested when ingested orally, and may not be able to penetrate the skin because of the large molecular size of collagen. Thus, a method for increasing the ability of the body to produce new collagen would have tremendous utility.

Shilajit, also known as "Moomiyo," is found in the high altitudes of the Himalayan Mountains and is considered as one of the "wonder medicines" of Ayurveda, the traditional Indian system of medicine dating back to 3500 B.C.E. Shilajit is regarded as one of the most important components in the Ayurvedic System of medicine and is also used as an adaptogen. S. Ghosal et al., Shilajit I: chemical constituents, 65 J. PHARM. SCI. 772 (1976); C. Velmurugan et al., Evaluation of safety profile of black shilajit after 91 days repeated administration in rats, 2 ASIAN PAC. J. TROP. BIOMED. 210 (2012). Shilajit is regarded as a "maharasa" (super-vitalizer) in Ayurveda. Shilajit is composed of rock humus, rock minerals, and organic substances that have been compressed by layers of rock mixed with marine organisms and microbial metabolites. Shilajit oozes out of the rocks in the Himalayas at higher altitudes ranging from 1000 to 5000 meters as black mass, as the rocks become warm during summer. C. Velmurugan et al., *supra* at 210. Shilajit contains fulvic acids ("FAs") as its main components, along with dibenzo-α-pyrones ("DBPs") and DBP chromoproteins, humic acid, and more than forty (40) minerals.

Natreon Inc.'s patented ingredient, PrimaVie^{®}, is a purified and standardized Shilajit extract for nutraceutical use. U.S. Patent Nos. 6,869,612 and 6,440,436. Research indicates that PrimaVie^{®} Shilajit has targeted action for increasing energy, revitalization, and anti-aging. It is standardized to have not less than sixty (60) percent fulvic acid and equivalents with high levels of dibenzo-α-pyrones and dibenzo-α-pyrone chromoproteins. Fulvic acid complex, derived from Shilajit, is an assembly of naturally occurring low and medium molecular weight compounds comprising oxygenated DBPs as the core nucleus, both in reduced as well as in oxidized form, and acylated DBPs and lipids as partial structural units, along with FAs. Thus, the active constituents of Shilajit contain DBPs and related metabolites, small peptides (constituting non-protein amino acids), some lipids, carrier molecules, and FAs. Ghosal et al., *supra* at 772; H. Meena et al., Shilajit: A panacea for high-altitude problems, 1 INT'L J. AYURVEDA RES. 37 (2010); S. Ghosal et al., Shilajit Part 7 ― Chemistry of Shilajit, an immunomodulatory ayurvedic rasayana, 62 PURE APPL. CHEM. (IUPAC) 1285 (1990); S. Ghosal et al., The core structure of Shilajit humus, 23 SOIL BIOL. BIOCHEM. 673 (1992).

Shilajit finds extensive use in Ayurveda, for diverse clinical conditions. For centuries, people living in the isolated villages in Himalaya and adjoining regions have used Shilajit alone, or in combination with other plant remedies, to prevent and combat problems with diabetes. Tiwari, V.P. et al., An interpretation of Ayurvedica findings on Shilajit, 8 J. RES. INDIGENOUS MED. 57 (1973). Moreover, being an antioxidant, Shilajit will prevent pancreatic islet cell damage, which is induced by cytotoxic oxygen radicals. Bhattacharya S.K., Shilajit attenuates streptozotocin induced diabetes mellitus and decrease in pancreatic islet superoxide dismutase activity in rats, 9 PHYTOTHER. RES. 41 (1995); Bhattacharya S.K., Effects of Shilajit on biogenic free radicals, 9 PHYTOTHER. RES. 56 (1995); and Ghosal, S., et al., Interaction of Shilajit with biogenic free radicals, 34B INDIAN J. CHEM. 596 (1995). It has been proposed that the derangement of glucose, fat, and protein metabolism during diabetes results in the development of hyperlipidemia. In one study, Shilajit produced significant beneficial effects in the lipid profile in rats. Trivedi N.A., et al., Effect of Shilajit on blood glucose and lipid profile in alloxan-induced diabetic rats, 36 INDIAN J. PHARMACOL. 373 (2004). Oral supplementation with Shilajit significantly improved physiological energy status in albino mice in a model of forced swimming test S. Bhattacharya et al., Beneficial effect of processed Shilajit on swimming exercise induced impaired energy status of mice, 1 PHARMACOLOGY ONLINE 817 (2009).

As discussed, Shilajit has been used to treat various ailments. It is also recommended as a performance enhancer. In addition to functioning as electrolytes and antioxidants, FAs are reported to elicit many important effects in the biological systems of plants and animals including humans, such as: (a) improvement of the bioavailability of minerals and nutrients; (b) detoxification of toxic substances including heavy metals; and (c) improvement of immune function.

Further, DBPs in Shilajit have been hypothesized to participate in the electron transport inside mitochondria, thus facilitating production of more ATP, leading to increased energy. S. Bhattacharya et al., Shilajit dibenzo-α-pyrones: Mitochondria targeted antioxidants, 2 PHARMACOLOGY ONLINE 690 (2009). Thus, Shilajit is found to increase energy, among other beneficial quantities.

However, as described below, the utility of Shilajit or its components for preserving the health of tissues and organs of mammals containing collagen, for treating collagen-related disorders, or for muscle building and regeneration is completely novel and of tremendous value to mammals, including humans. Thus there is a need for such uses of Shilajit.

### SUMMARY OF THE INVENTION

The present invention offers such a way of increasing the ability of the body to produce collagen by up-regulating the genes involved in the synthesis of collagen and associated proteins by administration of Shilajit, thus helping to improve the health of skin, cartilage, tendons, connective tissues, muscles, vascular tissue, bone, and teeth. Such efficacy of Shilajit may be attributed to Shilajit as a whole, or its individual components: fulvic acid, 3-OH-dibenzo-α-pyrone, 3,8-dihydroxy-dibenzo-α-pyrone, dibenzo-α-pyrone chromoproteins, humic acid, and more than 40 minerals, or a combination of two or more of these components. Shilajit is also an antioxidant with an ORAC value of 2,300 µmoles TE/g, and this property may also be contributing to its efficacy.

An objective of the present invention is to offer a method of using Shilajit or its individual components, or a combination of two or more of these components, to induce the body of a mammal, including that of a human, to synthesize new collagen and/or related proteins, thus promoting the health of all of the tissues and organs containing collagen, including skin, connective tissue, muscle, cartilage, bone, and teeth, improve muscle building and regeneration, and/or treat collagen-related disorders.

### BRIEF DESCRIPTION OF THE FIGURES

FIG. 1 shows, in one embodiment of the present invention, the effect of PrimaVie^{®} Shilajit supplementation on the lipid profile in skeletal muscles of sedentary pre-obese to obese humans.
FIG. 2 shows, in one embodiment of the present invention, the effect of PrimaVie^{®} Shilajit supplementation on plasma creatine kinase, myoglobin, and plasma glucose in skeletal muscles of sedentary pre-obese to obese humans.
FIG. 3 shows, in one embodiment of the present invention, the effect of PrimaVie^{®} Shilajit supplementation on gene expression of different Collagens (I, V, VI, XIV) in skeletal muscles of sedentary pre-obese to obese humans after 8 weeks of supplementation.
FIG. 4 shows, in one embodiment of the present invention, the effect of PrimaVie^{®} Shilajit supplementation on FN1, TNXB, MYOF, and DCN gene expression in skeletal muscles of sedentary pre-obese to obese humans after 8 weeks of supplementation.
FIG. 5 shows, in one embodiment of the present invention, the effect of PrimaVie^{®} Shilajit supplementation on ELN, FBN1, HSD17B11, and HSD17B6 gene expression in skeletal muscles of sedentary pre-obese to obese humans after 8 weeks of supplementation.
FIG. 6 shows, in one embodiment of the present invention, the effect of PrimaVie^{®} Shilajit supplementation on different collagen gene expression in skeletal muscles of sedentary pre-obese to obese humans after 12 weeks of supplementation.
FIG. 7 shows, in one embodiment of the present invention, the effect of PrimaVie^{®} Shilajit supplementation on FN1, TNXB, MYOF, and DCN gene expression in skeletal muscles of sedentary pre-obese to obese humans after 12 weeks of supplementation.
FIG. 8 shows, in one embodiment of the present invention, the effect of PrimaVie^{®} Shilajit supplementation on ELN, FBN1, HSD17B11, and HSD17B6 gene expression in skeletal muscles of sedentary pre-obese to obese humans after 12 weeks of supplementation.
FIG. 9 depicts a high performance liquid chromatogram (HPLC) of PrimaVie^{®} Shilajit using a RP-C 18 column.
FIG. 10 shows, in one embodiment of the present invention, the changes in lipid profile following PrimaVie^{®} Shilajit supplementation and exercise in skeletal muscles of healthy overweight/Class 1 obese human subjects.
FIG. 11 shows, in one embodiment of the present invention, the changes in blood glucose and muscle damage markers creatine kinase and myoglobin, following PrimaVie^{®} Shilajit supplementation and exercise, in the skeletal muscles of healthy overweight/Class 1 obese human subjects.
FIG. 12 shows, in one embodiment of the present invention, the effect of PrimaVie^{®} Shilajit supplementation on different types of collagen gene expression in skeletal muscles of healthy overweight/Class 1 obese human subjects.
FIG. 13 shows, in one embodiment of the present invention, the effect of PrimaVie^{®} Shilajit supplementation on FN1, TNXB, MYOF, and DCN gene expression in skeletal muscles of healthy overweight/Class 1 obese human subjects.
FIG. 14 shows, in one embodiment of the present invention, the effect of PrimaVie^{®} Shilajit supplementation on ELN, FBN1, HSD17B11, and HSD17B6 gene expression in skeletal muscles of healthy overweight/Class 1 obese human subjects.

### DETAILED DESCRIPTION

In one embodiment of the present invention, the method of promoting tissue and organ health, improving muscle building, and regenerating and/or treating collagen-related disorders comprises administering a dose of Shilajit between about 20 milligrams and about 2000 milligrams per day to a human subject.

In an alternative embodiment of the present invention, the method of promoting tissue and organ health, improving muscle building, and regenerating and/or treating collagen-related disorders comprises administering a dose of Shilajit between about 100 milligrams and about 500 milligrams per day to a human subject.

In order to determine if Shilajit would induce the body of a human to produce new collagen, a human clinical study was conducted. The study was aimed at determining the effect of Shilajit supplementation and exercise training on human skeletal muscle adaptation in a group of overweight/class 1 obese human subjects within twelve weeks of total study period. The hypothesis of the study was that oral supplementation of Shilajit would influence ECM-associated gene expression in the skeletal muscle and also that oral intake would synergistically act with treadmill exercise training to favorably impact skeletal muscle gene expression.

### 1. Materials and methods:

Test product, PrimaVie^{®} Shilajit Capsules, 250 mg, were supplied by Natreon, Inc., 2D Janine Place, New Brunswick, NJ 08901. The capsules contained gelatin, microcrystalline cellulose, croscarmellose sodium, fumed silicon-dioxide, and magnesium stearate as excipients, which are of NF grade.

### 1.1 High performance liquid chromatogram (HPLC) analysis of PrimaVie^{®} Shilajit

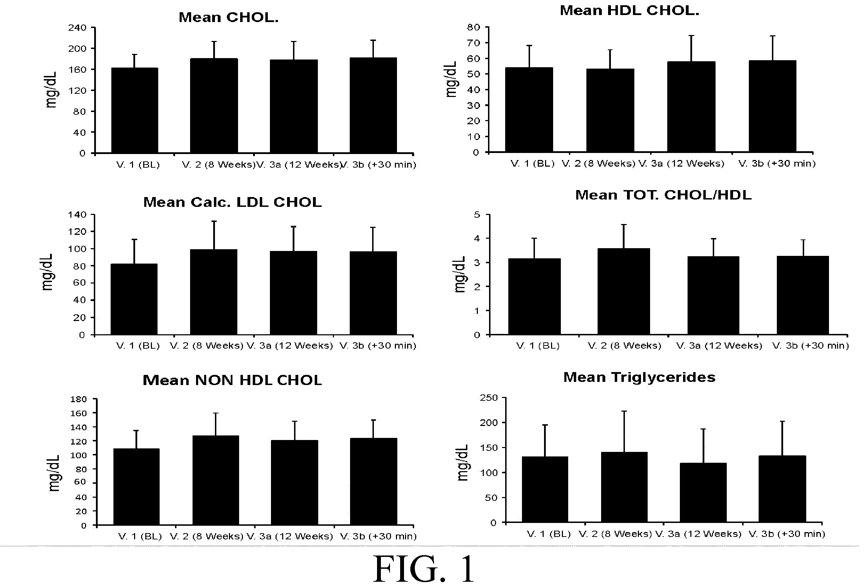

PrimaVie^{®} Shilajit is a purified and standardized Shilajit, containing not less than 0.3% DBPs, not less than 10.0% DBP chromoproteins, and not less than 50% FAs having DBP core nucleus and manufactured by a process to reduce the heavy metals to less than 1 ppm of lead, less than 1 ppm of arsenic, and less than 0.1 ppm of mercury. Quality control is done by high performance liquid chromatogram (HPLC). HPLC analysis was performed under ambient conditions, with Waters HPLC equipment comprising Waters 515 pumps, Waters photodiode array detector (PDA) model 2996, Waters pump controller module and Empower software (Version 1). Samples (20 µL) were injected by means of a Rheodyne injector fitted with a 20 µL loop. Compounds were separated on a C18 reverse-phase (Lichocart^{®} column (250 mm X 4 mm, i.d., 5-µm particle; column no. 331303, Darmstadt, Germany)) using the mobile phase Water:Acetonitrile:o-Phosphoric Acid [67:32:01 v/v/v] with a flow rate of 0.8 mL/min. The UV absorbance of eluent was monitored at a wavelength of 240 nm. The HPLC profile of the purified PrimaVie^{®} Shilajit and the chromatographic conditions is shown in FIG. 1.

### 1.2 Study subjects and experimental design:

The Western Institutional Review Boards ("WIRB") approved the study protocols (clinicaltrials.gov NCT02026414) and materials. All subjects provided written informed consent before participation in the study.

Overweight/Class 1 obese (body-mass index [BMI]: 25-35) adult (21-70 years) healthy human subjects of both genders were eligible to participate in this study. Participants were asked to fast overnight before blood sample collections. Any self-reported deviations in diet or exercises were recorded. The subjects were excluded from the study if any one of the following medications was used for management/treatment of CVD-related disorders: steroids (Prednisone, etc.), beta-blockers, hydrochlorothiazide, statins (Crestor, Lipitor, etc.), aspirin, and ACE inhibitors. Pregnant females as well as individuals who were therapeutically immunocompromised were also excluded from the study. The demographics of subjects participating in this study are presented in Table 1. The study design included three follow-up visits during the 12-week study period following their first initial baseline visit. The first follow-up visit was eight weeks after the patient received the supplement. The second follow-up visit was after four weeks of supplementation and exercise following the first follow-up visit, and the third follow-up visit was on the same day, thirty minutes post-exercise. At each visit, 50 mL of blood, 5-mm muscle biopsy, and demographic information including age, sex, height, weight, BMI, blood pressure, and pulse were taken. *See* Table 1.

**TABLE 1**

| Table 1: Demographic characteristics of study participants.* | | | | |
|---|---|---|---|---|
| Parameters | Mean Values | Baseline | 8 weeks | 12 weeks |
| Subjects (n) | 16 | - | - | - |
| Age (years) | 35.7±3.4 | - | - | - |
| Gender | - | - | - | - |
| Male | 6 | - | - | - |
| Female | 10 | - | - | - |
| Body Weight (lb.) | - | 188.5±8.6 | 187.9±8.8 | 187.4±8.9 |
| Body-mass index, kg/m² | - | 28.9±0.6 | 28.8±0.7 | 29.3±0.7 |
| Blood pressure systolic, mm Hg | - | 114.3±3.1 | 114.8±2.8 | 112.1±2.5 |
| Blood pressure diastolic, mm Hg | - | 74±1.9 | 71.6±1.5 | 69.8±1.8 |
| Pulse (min) | - | 70.1±2.0 | 72.4±1.9 | 70.8±2.2 |

| | | | | |
|---|---|---|---|---|
| *Values are expressed as mean ± SEM | | | | |

### 1.3 Supplementaiton regiment and compliance:

Each subject received 250 mg of PrimaVie^{®} Shilajit (Natreon, Inc.) twice a day for the first 8 weeks they were enrolled in the study. For the last 4 weeks of the study, subjects took 250 mg of PrimaVie^{®} Shilajit (Natreon, Inc.) supplement twice a day while also completing exercise on a treadmill (70-75% of maximum heart rate exercise for 20 minutes, plus 5 minutes of warm up and 5 minutes of cool down exercises, for a total of 30 minutes a day, 3 days a week). All subjects participated for a total of 12 weeks of the study, including three follow-up visits and one baseline visit.

### 1.4 Safety monitoring:

No adverse effect directly related to the dietary supplement was reported by clinical research staff.

### 1.5 Blood sampling and laboratory methods:

At all baseline and follow-up visits, peripheral venous blood was collected in heparinized tubes and transported on ice immediately to assess lipid profile, glucose, creatine kinase ("CK"), and serum myoglobin levels. Among lipid profile total cholesterol ("HDL"), high-density lipoprotein cholesterol ("HDL-C"), low density lipoprotein cholesterol ("LDL-C"), and triglyceride levels, calculated LDL cholesterol and non-HDL cholesterol levels were measured using standard clinical lipid profiles, and creatine kinase, glucose, and serum myoglobin tests were done at the Ohio State University Wexner Medical Center Clinical Laboratories, following manufacturer's instructions. During this study, whole blood was centrifuged at 4227 RCF for 3 minutes at 4°C to separate plasma. Plasma was aliquoted and stored at -80°C for further analysis.

### 1.6 Muscle biopsy collection

Biopsy site: vastus lateralis or gastrocnemius. A biopsy was collected by a board-certified physician after application of local anesthetics to the site of biopsy using a 100-120V, 50-60Hz, 600VA biopsy machine having 12-gauge SenoRx, stereotactic ultrasound Encor Probe (BARD Encor Ultra, breast biopsy system; AZ, USA). Muscle samples were used for mRNA expression profiling and RT-PCR.

### 1.7 GeneChip Probe Array Analyses

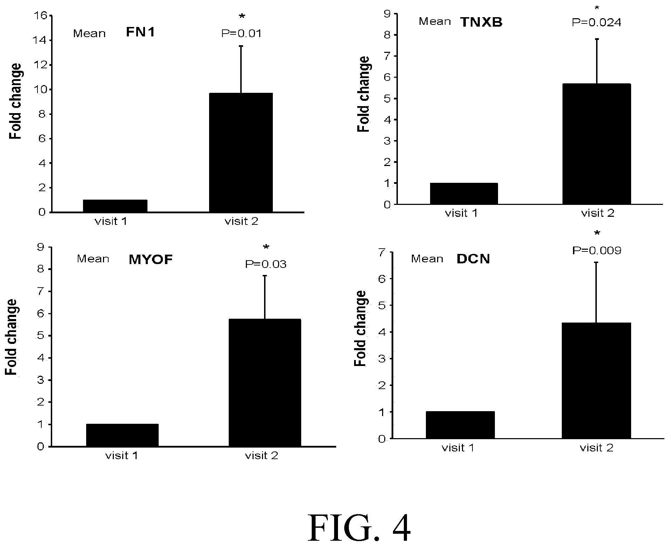

To identify sets of genes differentially expressed in the muscle samples of different time periods, RNA extraction, target labeling, GeneChip probe array analyses were performed using Affymetrix Human Transcriptome Array 2.0. Briefly, GeneChip IVT Labeling Kit (Affymetrix, Santa Clara, CA) in vitro transcription ("IVT") reaction was used to generate biotinylated cRNA from RNA samples. The samples were hybridized, the arrays were washed, stained with streptavidinphycoerythrin and scanned with a Gene Array scanner (Affymetrix). Gene Chip Operating Software ("GCOS," Affymetrix) was employed for data acquisition and image processing. Raw data were analyzed using Genespring GX (Agilent, Santa Clara CA). Additional processing of data was performed using dChip software (Harvard University). GC-RMA (Gene Spring GX, Agilent, Santa Clara, CA) was applied for data normalization. Differentially expressed genes were identified using a two-class *t*-test where significance level was set at *p*<0.05 with Benjamin Hochberg correction for false discovery rate. The genes that were significantly upregulated were subject to functional analysis using the Database for Annotation, Visualization, and Integrated Discovery ("DAVID," NIAID, NIH), and gene ontology ("GO"). ECM-associated genes were identified as a major cluster that changed following Shilajit supplementation (Table 4). Selected differentially-expressed candidate genes were verified using quantitative real-time PCR (FIGS. 4-6).

### 1.8 Validation of microarray results using quantitative real-time PCR

Real-time PCR was carried out to verify the candidate mRNAs revealed by microarray. Levels of selected genes were assayed by real-time PCR using double-stranded DNA-binding dye SYBR Green-I. GAPDH was used as a reference housekeeping gene.

### 1.9 Data Analysis

### Statistical methods:

Multivariate linear regression was used to test if all 14 gene expression (ΔΔCT) values were jointly different across adjacent time points. Five comparisons were generated across the various time points. The multivariate regression produces estimated differences along with their 95% confidence interval for each gene with a single *p*-value, testing if all 14 genes' ΔΔCT values were jointly different across adjacent time points. Multivariate regression was used because the study was not powered to run 28 individual comparisons. Multivariate normality was checked using standardized normal probability plots. If any values were not normal, then they were transformed using natural logarithms. A new multivariate linear regression model was used to check if patient lipids/glucose/muscle damage marker values were jointly different across the adjacent time points. Lipids/glucose/muscle damage marker values were summarized using means and standard deviations for each of the three time points. All analyses were run using Stata 13.1, StataCorp, College Station, TX.

### 2. Results

### 2.1 Changes in glucose, lipid profile, creatine kinase, and serum myoglobin levels following PrimaVie^{®} Shilajit supplementation:

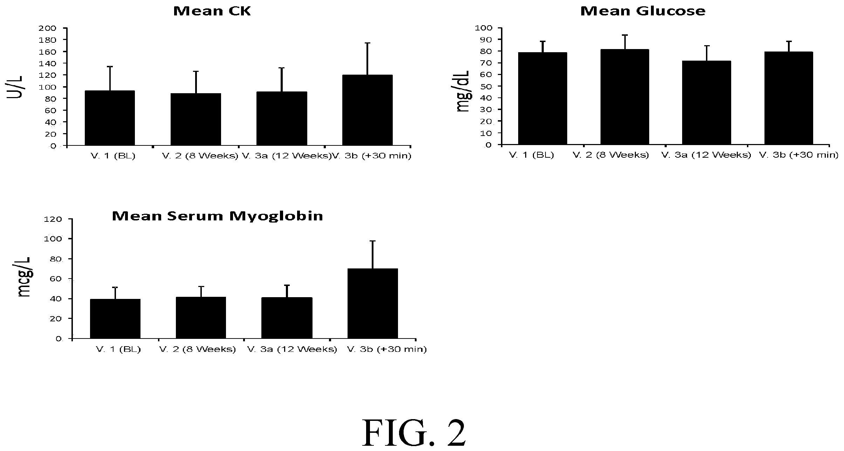

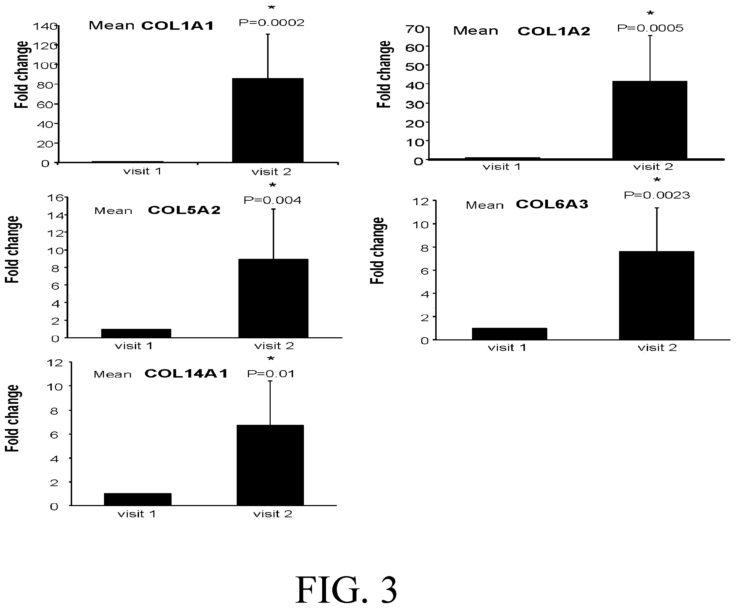

Lipid profile measurements indicated good toleration without any significant changes in the mean cholesterol, mean HDL cholesterol, mean calculated LDL cholesterol, mean total cholesterol/HDL, mean non-HDL cholesterol, and triglycerides following 8 weeks of PrimaVie^{®} Shilajit supplementation compared to the levels observed during baseline visits (FIG. 2, Tables 2 and 3). Additionally, lipid profile levels after 12 weeks of supplementation period with exercise and thirty-minute post-final exercise on the same day remained unchanged compared to the levels observed during baseline and 8-week visits (FIG. 2, Tables 2 and 3). Further, no adverse changes were observed in other variables, such as blood glucose and muscle damage markers, including creatine kinase and serum myoglobin levels, at any follow-up visits (FIG. 3, Tables 2 and 3).

**TABLE 2**

| Table 2: Summary statistics of lipids, glucose, and muscle damage markers of baseline, 8-weeks, and 12-weeks (pre- and post-final exercise) visits, based on a linear regression model* | | | | | | | | | | |
|---|---|---|---|---|---|---|---|---|---|---|
| Variables | CK | Gluc. | CHOL | Triglyc. | HDL | Calc. LDL | Total CHOL/ | CHOL (Non-LDL | Myo. HDL) | |
| BL | N | 16 | 16 | 16 | 16 | 16 | 15 | 16 | 16 | 16 |
| | Mean | 121.50 | 79.56 | 175.06 | 4.77 | 52.06 | 96.27 | 3.51 | 123.00 | 40.38 |
| | SD | 80.48 | 11.04 | 28.48 | 0.52 | 12.22 | 31.96 | 0.92 | 30.58 | 12.12 |
| 8 weeks | N | 16 | 16 | 16 | 16 | 16 | 15 | 16 | 16 | 15 |
| | Mean | 92.13 | 80.88 | 187.38 | 4.88 | 52.00 | 106.38 | 3.74 | 135.38 | 40.06 |
| | SD | 38.57 | 12.03 | 34.46 | 0.43 | 11.14 | 33.53 | 0.96 | 34.67 | 9.89 |
| 12 weeks (pre) | N | 16 | 16 | 16 | 16 | 16 | 15 | 16 | 16 | 15 |
| | Mean | 118.69 | 77.50 | 184.00 | 4.74 | 55.44 | 103.07 | 3.46 | 128.56 | 42.40 |
| | SD | 73.84 | 15.52 | 34.59 | 0.57 | 15.22 | 30.03 | 0.84 | 30.25 | 12.65 |
| 12 weeks (post) | N | 16 | 16 | 16 | 16 | 16 | 15 | 16 | 16 | 15 |
| | Mean | 142.19 | 82.56 | 184.50 | 4.82 | 55.31 | 101.00 | 3.47 | 129.19 | 66.13 |
| | SD | 81.12 | 11.54 | 33.05 | 0.52 | 14.69 | 28.81 | 0.80 | 29.02 | 26.72 |

| | | | | | | | | | | |
|---|---|---|---|---|---|---|---|---|---|---|
| *Gluc., glucose; CHOL, Cholesterol; LDL, low-density lipoprotein; HDL, high-density lipoprotein; Triglyc., triglycerides; Myo., myoglobin; calc., calculated; pre, pre-final exercise; post, post-final exercise. | | | | | | | | | | |

**TABLE 3**

| Table 3: Multivariate test of all lipids/glucose/muscle damage markers (jointly) for all comparisons requested | | | | |
|---|---|---|---|---|
| Comparison | Contrast | Std. Err. | Z | *p-value* |
| 8 weeks vs. BL | -14.01 | 16.64 | -0.84 | 0.400 |
| 9-12 weeks AM vs. BL | -9.78 | 17.20 | -0.57 | 0.570 |
| 9-12 weeks AM vs. 8 weeks | 4.43 | 16.94 | 0.25 | 0.803 |
| 9-12 weeks PM vs. 8 weeks | 26.80 | 16.94 | 1.58 | 0.114 |
| 9-12 weeks PM vs. 9-12 weeks AM | 22.57 | 17.49 | 1.29 | 0.197 |

### 2.2 Microarray analysis of skeletal muscle following PrimaVie^{®} Shilajit supplementation

To determine the changes in mRNA expression profile in response to supplementation and exercise, muscle samples were collected during each visit, and RNA extraction, target labeling, and GeneChip data analysis were performed using Affymetrix Human Transcriptome Array 2.0 as described previously. The genes that were expressed highly in the skeletal muscle samples after 8 weeks of PrimaVie^{®} Shilajit supplementation, compared to baseline visits, were selected for further studies. Table 4 shows the top 12 genes that were up-regulated in the muscles during 8 weeks of PrimaVie^{®} Shilajit supplementation compared to baseline visits. Those significantly up-regulated genes are tenascin XB (TNXB), decorin (DCN), myoferlin (MYOF), collagen (COL) (type I, II, III, V, VI, XIV), elastin (ELN), fibrillin 1 (FBN1), and fibronectin 1 (FN1) (Table 4).

**TABLE 4**

| Table 4: List of genes significantly up-regulated in skeletal muscles of healthy overweight/Class 1 obese humans following 8 weeks of PrimaVie^{®} Shilajit supplementation | | | | |
|---|---|---|---|---|
| gene description | gene symbol | regulation | fold change | p value* |
| Tenascin XB | TNXB | Up | 1.78 | 0.0311 |
| Tenascin XB | TNXB | Up | 1.76 | 0.0467 |
| Tenascin XB | TNXB | Up | 1.75 | 0.0425 |
| Tenascin XB | TNXB | Up | 1.74 | 0.0427 |
| Tenascin XB | TNXB | Up | 1.71 | 0.0430 |
| Decorin | DCN | Up | 2.23 | 0.0186 |
| Decorin | DCN | Up | 1.09 | 0.0338 |
| Myoferlin | MYOF | Up | 1.11 | 0.0207 |
| Collagen I alpha-1 | COL1A1 | Up | 4.61 | 0.014905 |
| Collagen I alpha-2 | COL1A2 | Up | 5.13 | 0.007683 |
| Collagen III alpha-1 \|microRNA3606 | COL3A1\|MIR36060 | Up | 5.18 | 0.008654 |
| Collagen V alpha-2 | COL5A2 | Up | 1.62 | 0.032423 |
| Collagen VI alpha-3 | COL6A3 | Up | 2.96 | 0.012206 |
| Collagen XIV alpha-1 | COL14A1 | Up | 2.07 | 0.006124 |
| Elastin | ELN | Up | 1.13 | 0.018775 |
| Fibrillin1 | FBN1 | Up | 3.05 | 0.025495 |
| Fibronectin1 | FN1 | Up | 3.65 | 0.00847 |

| | | | | |
|---|---|---|---|---|
| *p < 0.05 | | | | |

### 2.3 Validation of microarray results using quantitative real-time PCR

To verify the accuracy of the microarray-based mRNA quantification, the above-mentioned up-regulated ECM-associated genes, and, additionally, the HSD17B11 and HSD17B6 genes, were re-examined using RT-PCR. Consistent with microarray results, RT-PCR results also revealed significant changes in the expression of those ECM-associated genes in the muscle samples after 8 weeks of PrimaVie^{®} Shilajit supplementation compared to the baseline visit (FIGS. 4-6 and Table 5) and 12 weeks of supplementation with exercise (before and after final exercise) compared to 8-week visits (FIGS. 4-6 and Table 5).

**TABLE 5**

| Table 5: Multivariate test of all genes (jointly) for all comparisons requested | | | | |
|---|---|---|---|---|
| Comparison | Contrast | Std. Err. | Z | *p-value* |
| 8 weeks vs. BL | -3.68 | 1.22 | -3.02 | 0.003 |
| 9-12 weeks AM vs. BL | -6.47 | 1.22 | -5.30 | < 0.001 |
| 9-12 weeks AM vs. 8 weeks | -2.79 | 1.22 | -2.29 | 0.022 |
| 9-12 weeks PM vs. 8 weeks | -2.56 | 1.22 | -2.10 | 0.035 |
| 9-12 weeks PM vs. 9-12 weeks AM | 0.22 | 1.22 | 0.18 | 0.855 |

### 3. Discussion and conclusion

Skeletal muscle represents the largest metabolically active tissue in the body and accounts for approximately 40% of body mass. It is an adaptive tissue that is composed of heterogeneous muscle fibers that differ in their contractile and metabolic profiles. The study was designed to determine the changes in blood lipid composition as well as alterations in muscle damage markers such as creatine kinase, serum myoglobin, and blood glucose in skeletal muscles of healthy overweight/Class 1 obese human subjects following 8 weeks of supplementation, an additional 4 weeks of supplementation with exercises, and 30 minutes post-exercise on the same day. Earlier, it was well-established that obesity is characterized by increases in circulating lipids (FFAs, triglycerides) that accumulate in muscle as triacylglycerol and fatty acid metabolites such as ceramide, diacylglycerol, and long chain acyl CoA. It was also observed that HDL-cholesterol levels are associated with other metabolism parameters, such as triglyceride and LDL-cholesterol levels. But in this study, no alterations in blood lipid profiles of healthy overweight/class 1 obese human subjects were reported following 8 weeks of PrimaVie^{®} Shilajit supplementation as well as the subsequent 4 weeks of supplementation with exercise, nor on the same day 30 minutes after final exercise, suggesting no adverse effect of PrimaVie^{®} Shilajit supplementation on skeletal muscle. Phosphocreatine ("PCr") is one major source of ATP replenishment in tissues with rapidly fluctuating energy demand. This supply is mediated by the creatine kinase ("CK") reaction, in which creatine ("Cr") and ADP are reversibly phosphorylated to PCr and ATP, respectively. The PCr-CK system, which functions as a spatial and temporal buffer of ATP levels, requires a high level of total cellular creatine in mammal skeletal muscle. Thus, reduction in creatine kinase may disturb ATP formation within skeletal muscle. High intracellular creatine concentrations are accomplished by a combination of endogenous production and exogenous dietary intake, followed by cellular uptake of creatine from blood vessels. In this study, no changes in serum creatine kinase level were observed after the first 8 weeks of supplementation, the next 4 weeks of supplementation with exercise, and in the third follow-up visit after 30 minutes post-exercise in healthy overweight/Class 1 obese human subjects, which provides evidence for an important role of PrimaVie^{®} Shilajit in maintaining skeletal muscle integrity. Myoglobin is mainly present in skeletal or cardiac muscle where its high concentration enables O₂ storage, in turn facilitating O₂ diffusion in cardiac and skeletal muscles. The unchanged levels of serum myoglobin in the human subjects following four visits also indicates the possible role of PrimaVie^{®} Shilajit in maintaining oxygen levels to the skeletal muscle under severe hypoxia. Besides these parameters, we also observed unaffected blood glucose level on all visits in healthy overweight/Class 1 obese human subjects. Taken together, this data suggests that PrimaVie^{®} Shilajit is well tolerated to physiological functions and maintains normal whole body glucose metabolism, homeostasis, and muscle integrity in the skeletal muscle of healthy human volunteers.

This is the first study to systematically assess the changes in expression of ECM-associated genes following PrimaVie^{®} Shilajit supplementation in healthy overweight/Class 1 obese human volunteers. In the present study, both microarray and RT-PCR results revealed elevated mRNA expression of different ECM components including different types of collagen, elastin, tenascin XB, decorin, myoferlin, fibrillin, fibronectin 1, HSD17B11, and HSD17B6 in the skeletal muscle following 8 weeks of PrimaVie^{®} Shilajit supplementation, 4 more weeks of supplementation with exercise, and 30 minutes post-exercise on the same day. Collagen maintains firmness and elasticity of the skin. Collagens I and III are formed in human skin in a higher proportion relative to other types and are maintained in a fixed proportion relative to one another in normal skin tissue. Collagen I constitutes about seventy (70) percent of collagen in the skin, with Collagen III constituting about ten (10) percent of collagen in the skin and Collagens IV, V, VI, and VII each constituting trace amounts of collagen in the skin. Collagen, in the form of collagen hydrolysate, keeps skin hydrated. Decrease in the amount of collagen in the body as we age results in sag, lines and wrinkles, lack of tension and elasticity, and delay in the wound-healing process. Previous studies demonstrated that increase of COL1A2, COL3A1, and COL5A1 gene expression helps in cellular proliferation and active remodeling of ECM during wound healing. Thus, increase in collagen expression in this study suggests the potential role of PrimaVie^{®} Shilajit in anti-aging. Although collagen provides the main structure, other ECM components are also playing an important role in skeletal muscle adaptation. Decorin, which is a small leucine-rich proteoglycan, also contributes to the formation and stabilization of collagen fibers in the perimysium that supports muscle fibers assembled with myogenesis. Myoferlin is highly expressed during muscle development, specifically in myoblasts undergoing fusion. In addition, fibronectin is a key protein that aids in the synthesis of provisional granulation tissue during early wound repair. Fibrillins, a type of micro fibril, is also one of the key structural elements in the ECM of skeletal muscle. They have been found ubiquitously distributed in connective tissues and are reported to be organized in tissue-specific architectures. In addition, the microfibril bundles were oriented parallel to each other and co-localized highly with elastin fibers. Another ECM component, Tenascin-X, determines the mechanical properties of collagen. Additionally, myoferlin has been shown to regulate the recycling of vascular endothelial growth factor receptor-2 (VEGFR-2). Myoferlin protein levels are normally low in adult skeletal muscle and nearly absent in healthy myofibers. It has been observed earlier that an increase in myoferlin level leads to an accumulation of mononuclear myoferlin-positive myoblasts that anxiously wait to repair damaged myofibers, suggesting that it is important during muscle regeneration.

In this study, collectively microarray and RT-PCR results showed high expression of these ECM-associated genes that have previously been shown to play positive roles in skeletal muscle development/regeneration and strongly recommended that PrimaVie^{®} Shilajit supplementation plays an important role in ECM fortification by maintaining skeletal muscle integrity and elasticity in adult healthy overweight/Class 1 obese human subjects.

It is apparent from Table 1 that Shilajit significantly up-regulated the genes responsible for the synthesis of collagen and other related proteins, especially Collagens I alpha-1, I alpha-2, and III alpha-1, which were up-regulated nearly 5-fold. Genes for Collagens V alpha-2, VI alpha-3, and XIV alpha-1 were up-regulated by 1.6-3 fold. No up-regulation of Collagen II was seen in this study because skeletal muscle, from which tissue samples were taken in this study, does not contain Collagen II.

Genes encoding tenascin, myoferlin, decorin, elastin, fibrillin, and fibronectin were also up-regulated.

All of the results were statistically significant with p values ranging from 0.006 to 0.05. The results are shown in FIGS. 1-14.

FIG. 1 shows the effect of PrimaVie^{®} Shilajit supplementation on the lipid profile in skeletal muscles of sedentary pre-obese to obese humans. Values are mean ± SEM (n=16). *denotes P < 0.05 compared to baseline. CHOL, cholesterol; HDL, high-density lipoprotein; LDL, low-density lipoprotein. There were no changes in the mean cholesterol, mean HDL cholesterol, mean calculated LDL cholesterol, mean total cholesterol/HDL, mean non-HDL cholesterol, and triglycerides following 8 weeks of PrimaVie^{®} Shilajit supplementation compared to the levels observed during baseline visits. Additionally, lipid profile levels after 12 weeks of supplementation period with exercise remained unchanged compared to the levels observed during average baseline visits.

FIG. 2 shows the effect of PrimaVie^{®} Shilajit supplementation on plasma creatine kinase, myoglobin, and plasma glucose in skeletal muscles of sedentary pre-obese to obese humans. Values are mean ± SEM (n=16). *denotes P < 0.05 compared to baseline. No adverse changes were observed in blood glucose, creatine kinase, and serum myoglobin levels after first 8 weeks of supplementation and at 12 weeks of supplementation with exercise, compared to the baseline visits.

FIG. 3 shows the effect of PrimaVie^{®} Shilajit supplementation on gene expression of different Collagens (I, V, VI, XIV) in skeletal muscles of sedentary pre-obese to obese humans. mRNA expression levels of COL1A1, COL1A2, COL5A2, COL6A3, and COL14A1 in muscle biopsies were measured using RT-PCR. The effect of PrimaVie^{®} supplementation (250mg/b.i.d) were measured during the course of the visit after 8 weeks of supplementation. Data are mean ± SEM (n=16); ^{∗}p < 0.05 compared to the baseline visit. COL1A1, Collagen I alpha 1; COL1A2, Collagen I alpha 2; COL5A2, Collagen V alpha 2; COL6A3, Collagen VI alpha 3; COL14A1, Collagen XIV alpha 1. RT-PCR results showed high expression of these genes in the muscle samples after 8 weeks of PrimaVie^{®} supplementation compared to the baseline visit. Collectively, high expression of these genes that have previously been shown to play positive roles in skeletal muscle development/regeneration and validation using RT-PCR strongly recommended that PrimaVie^{®} supplementation plays an important role in muscle adaption/regeneration.

FIG. 4 shows the effect of PrimaVie^{®} Shilajit supplementation on FN1, TNXB, MYOF, and DCN gene expression in skeletal muscles of sedentary pre-obese to obese humans. mRNA expression levels of FN1, TNXB, MYOF, and DCN in muscle biopsies were measured using RT-PCR. The effect of PrimaVie^{®} supplementation (250 mg/b.i.d) were measured during the course of visit after 8 weeks of supplementation. Data are mean ± SEM (n=16); ^{∗}p < 0.05 compared to the baseline visit. FN, fibronectin; TNXB, tenascin XB; MYOF, myoferlin; DCN, decorin. RT-PCR results showed high expression of these genes in the muscle samples after 8 weeks of PrimaVie^{®} supplementation compared to the baseline visit. Collectively, high expression of these genes that have previously been shown to play positive roles in skeletal muscle development/regeneration and validation using RT-PCR strongly recommended that PrimaVie^{®} supplementation plays an important role in muscle adaption/regeneration.

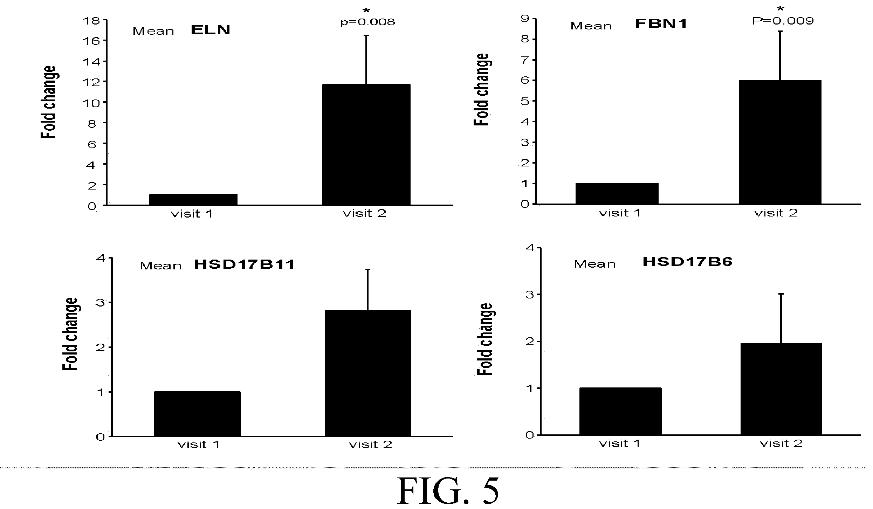

FIG. 5 shows the effect of PrimaVie^{®} Shilajit supplementation on ELN, FBN1, HSD17B11, and HSD17B6 gene expression in skeletal muscles of sedentary pre-obese to obese humans. mRNA expression levels of ELN, FBN1, HSD17B11, and HSB17B6 in muscle biopsies were measured using RT-PCR. The effect of PrimaVie^{®} supplementation (250mg/b.i.d) were measured during the visit after 8 weeks of supplementation. Data are mean ± SEM (n = 16); ^{∗}p < 0.05 compared to the baseline visit. ELN, elastin; FBN, fibrillin; HSD17B11, hydroxysteroid (17-Beta) dehydrogenase 11; HSD17B6, hydroxysteroid (17-Beta) dehydrogenase 6. RT-PCR results showed high expression of these genes in the muscle samples after 8 weeks of PrimaVie^{®} supplementation compared to the baseline visit. Collectively, high expression of these genes that have previously been shown to play positive roles in skeletal muscle development/regeneration and validation using RT-PCR strongly recommended that PrimaVie^{®} supplementation plays an important role in muscle adaption/regeneration.

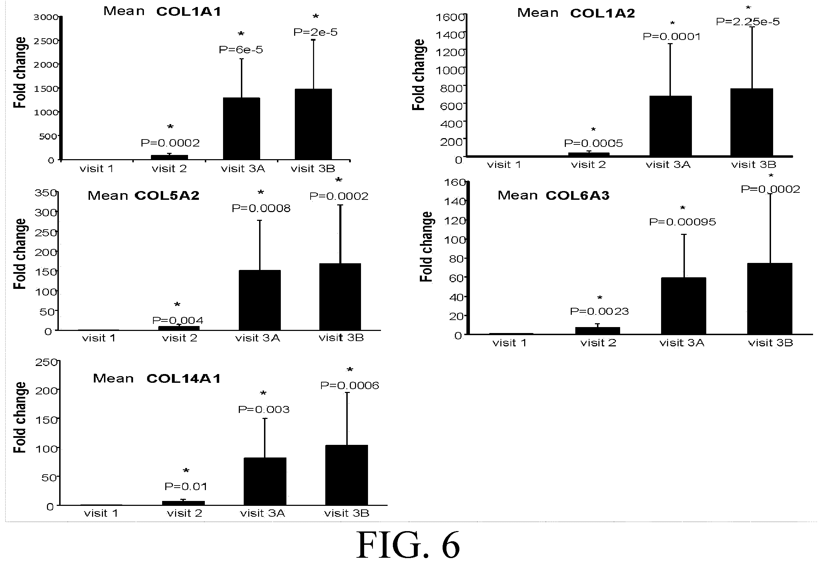

FIG. 6 shows the effect of PrimaVie^{®} Shilajit supplementation on different collagen gene expression in skeletal muscles of sedentary pre-obese to obese humans. mRNA expression levels of COL1A1, COL1A2, COL5A2, COL6A3, and COL14A1 in muscle biopsies were measured using RT-PCR. The effects of PrimaVie^{®} supplementation (250mg/b.i.d) were measured during the course of the entire visit: 8 weeks of supplementation, 12 weeks of supplementation with exercise (immediately before and after the final exercise routine). Data are mean ± SEM (n = 16); ^{∗}p < 0.05 compared to the baseline visit. COL1A1, Collagen I alpha 1, COL1A2, Collagen I alpha 2; COL5A2, Collagen V alpha 2; COL6A3, Collagen VI alpha 3; COL14A1, Collagen XIV alpha 1. RT-PCR results showed high expression of these genes in the muscle samples after 12 weeks of PrimaVie^{®} supplementation with exercise compared to the baseline visit. Collectively, high expression of these genes that have previously been shown to play positive roles in skeletal muscle development/regeneration and validation using RT-PCR strongly recommended that PrimaVie^{®} supplementation plays an important role in muscle adaption/regeneration.

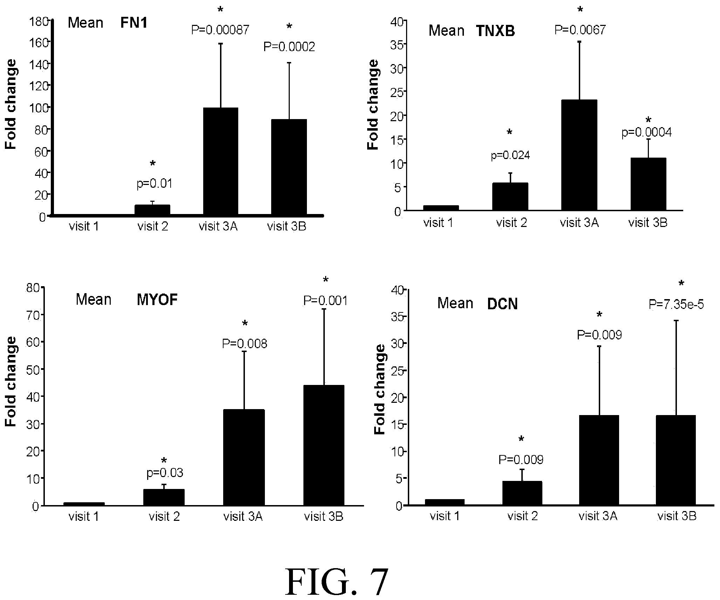

FIG. 7 shows the effect of PrimaVie^{®} Shilajit supplementation on FN1, TNXB, MYOF, and DCN gene expression in skeletal muscles of sedentary pre-obese to obese humans. mRNA expression levels of FN1, TNXB, MYOF, and DCN in muscle biopsies were measured using RT-PCR. The effect of PrimaVie^{®} supplementation (250 mg/b.i.d) were measured during the course of the visit: 8 weeks of supplementation, 12 weeks of supplementation with exercise (immediately before and after the final exercise routine). Data are mean ± SEM (n = 16); ^{∗}p < 0.05 compared to the baseline visit. FN, fibronectin; TNXB, tenascin XB; MYOF, myoferlin; DCN, decorin.

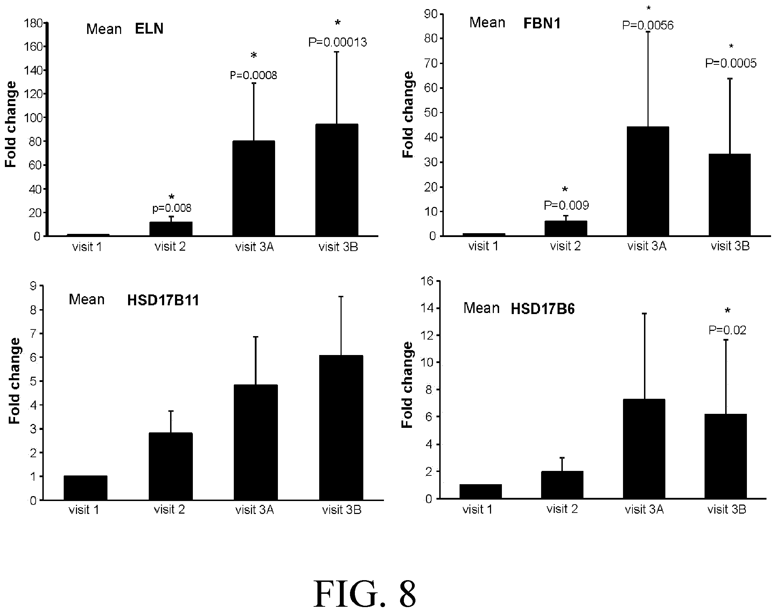

FIG. 8 shows the effect of PrimaVie^{®} Shilajit supplementation on ELN, FBN1, HSD17B11, and HSD17B6 gene expression in skeletal muscles of sedentary pre-obese to obese humans. mRNA expression levels of ELN, FBN1, HSD17B11, and HSB17B6 in muscle biopsies were measured using RT-PCR. The effect of PrimaVie^{®} supplementation (250 mg/b.i.d) were measured during the course of the entire visit: 8 weeks of supplementation, 12 weeks of supplementation with exercise (immediately before and after the final exercise routine). Data are mean ± SEM (n = 16); ^{∗}p < 0.05 compared to the baseline visit. ELN, elastin; FBN, fibrillin; HSD17B11, hydroxysteroid (17-Beta) dehydrogenase 11; HSD17B6, hydroxysteroid (17-Beta) dehydrogenase 6.

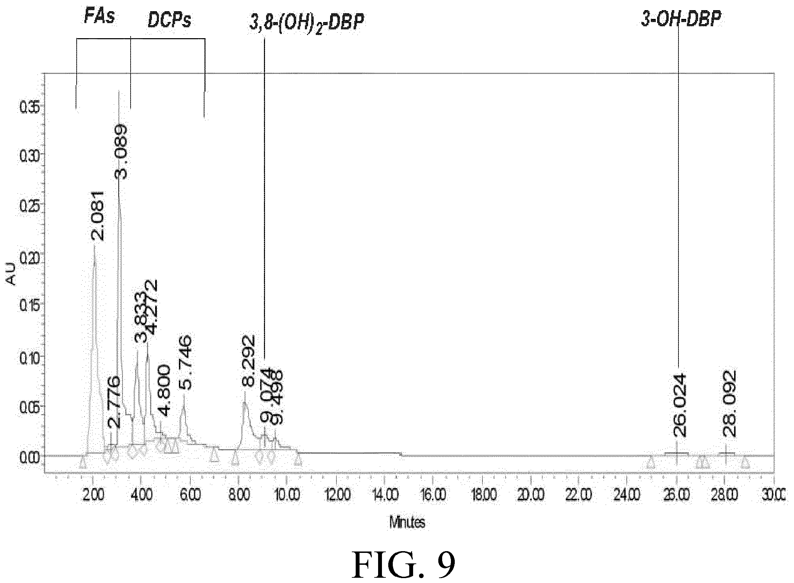

FIG. 9 is a high performance liquid chromatogram (HPLC) of PrimaVie^{®} Shilajit by RP-C 18 column. Fulvic acds (FAs) t_{R}: 2.0-3.0 min; Dibenzochromo proteins (DCPs) t_{R}: 3.0-5.8 min; 3,8-(OH)₂-Dibenzo-α-pyrone t_{R}: 9.07 min; 3-OH-Dibenzo-α-pyrone t_{R}: 26.02 min.

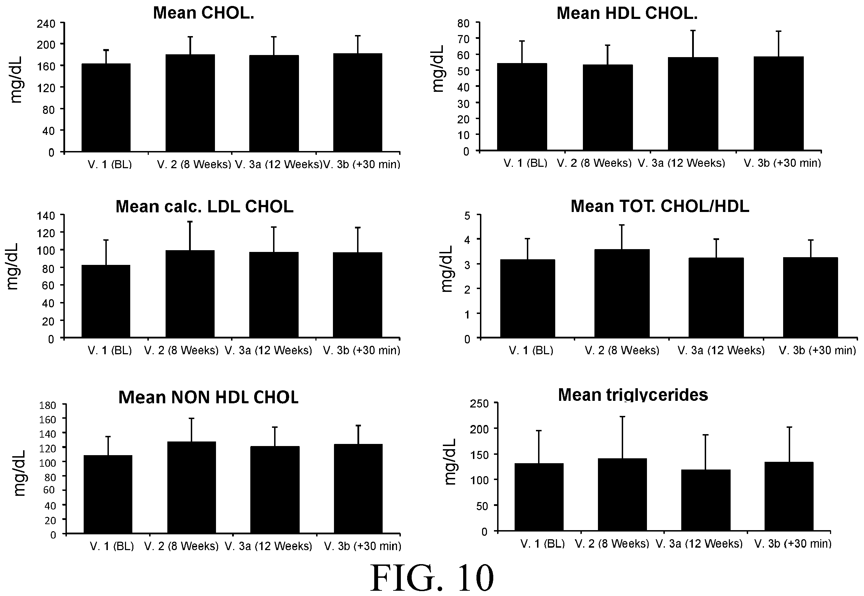

FIG. 10 shows the changes in lipid profile following PrimaVie^{®} Shilajit supplementation and exercise in skeletal muscles of healthy overweight/class I obese human subjects. The effects of PrimaVie^{®} Shilajit supplementation (250 mg/b.i.d) were measured during the course of the entire follow-up visit: 8 weeks of supplementation, next 4 weeks of supplementation with exercise (immediately before and after the 30-minute final exercise routine). Values are mean ± SEM (n = 16). (CHOL, cholesterol; HDL, high-density lipoprotein; LDL, low-density lipoprotein).

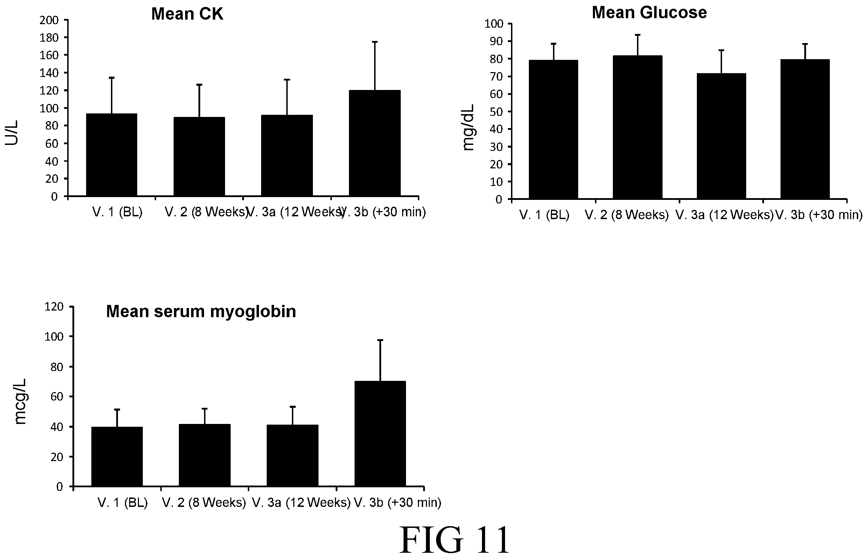

FIG. 11 shows the changes in blood glucose and muscle damage markers creatine kinase and myoglobin, following PrimaVie^{®} Shilajit supplementation and exercise, in the skeletal muscles of healthy overweight/Class 1 obese human subjects. The effects of PrimaVie^{®} Shilajit supplementation (250 mg/b.i.d) were measured during the course of the entire follow-up visit: 8 weeks of supplementation, next 4 weeks of supplementation with exercise (immediately before and after the 30-minute final exercise routine). Values are mean ± SEM (n=16).

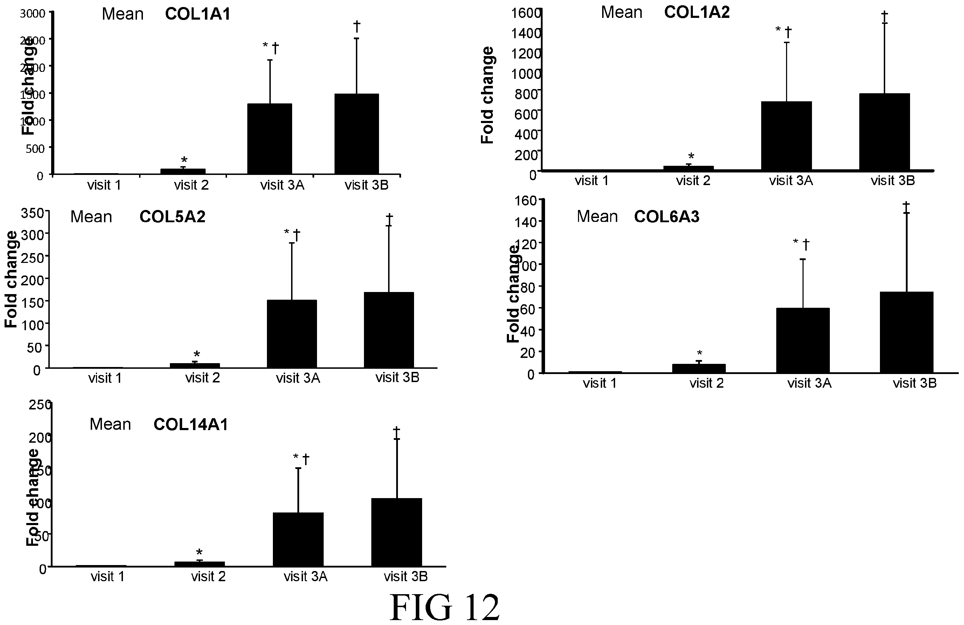

FIG. 12 shows the effect of PrimaVie^{®} Shilajit supplementation on different types of collagen gene expression in skeletal muscles of healthy overweight/Class 1 obese human subjects. mRNA expression levels of COL1A1, COL1A2, COL5A2, COL6A3, and COL14A1 were measured using RT-PCR from muscle biopsies. The effects of PrimaVie^{®} Shilajit supplementation (250mg/b.i.d) were measured during the course of the entire visit: 8 weeks of supplementation, then 4 additional weeks of supplementation with exercise (immediately before and after 30-minute final exercise routine). Data are mean ± SEM (n=16); ^{∗}p < 0.005 compared to the baseline visit and †p < 0.05 compared to the 8-weeks visit. No significant changes were observed between the 12-weeks pre- and post-30-minute final exercise periods. Visit 1, baseline visit; Visit 2, after 8 weeks of supplementation; Visit 3A, after 12 weeks of supplementation with exercise (before 30 minutes of final exercise); Visit 3B, after 12 weeks of supplementation with exercise (after 30 minutes of final exercise); COL1A1, Collagen I alpha-1; COL1A2, collagen type I alpha-2; COL5A2, Collagen V alpha-2; COL6A3, Collagen VI alpha-3; COL14A1, Collagen XIV alpha-1.

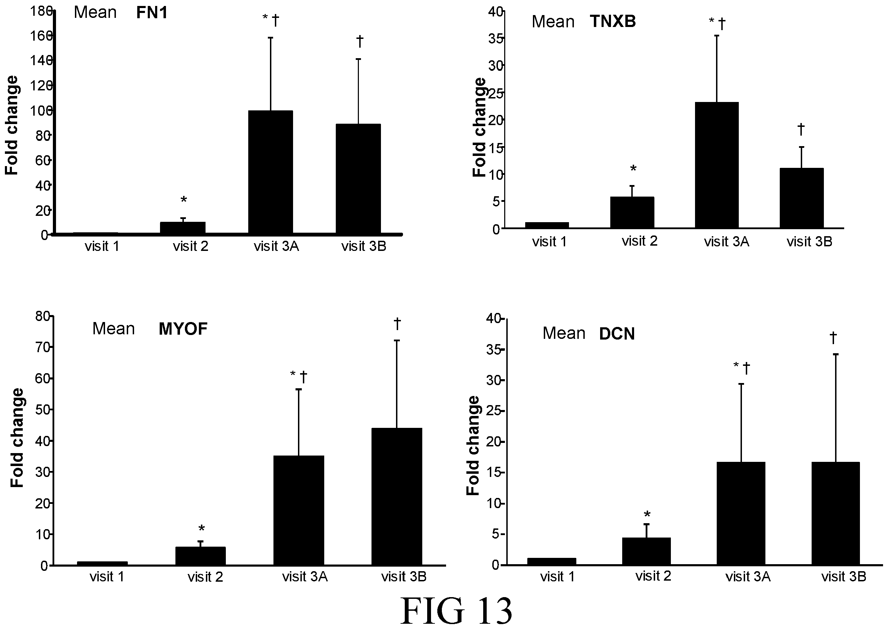

FIG. 13 shows the effect of PrimaVie^{®} Shilajit supplementation on FN1, TNXB, MYOF, and DCN gene expression in skeletal muscles of healthy overweight/Class 1 obese human subjects. mRNA expression levels of FN1, TNXB, MYOF, and DCN were measured using RT-PCR from muscle biopsies. The effects of PrimaVie^{®} Shilajit supplementation (250mg/b.i.d) were measured during the course of the entire visit: 8 weeks of supplementation, then 4 additional weeks of supplementation with exercise (immediately before and after 30-minute final exercise routine). Data are mean ± SEM (n=16); ^{∗}p < 0.005 compared to the baseline visit and †p < 0.05 compared to the 8-weeks visit. No significant changes were observed between the 12-weeks pre- and post-30-minute final exercise periods. Visit 1, baseline visit; Visit 2, after 8 weeks of supplementation; Visit 3A, after 12 weeks of supplementation with exercise (before 30 minutes of final exercise); Visit 3B, after 12 weeks of supplementation with exercise (after 30 minutes of final exercise); FN1, fibronectin 1; TNXB, tenascin XB; MYOF, myoferlin; DCN, decorin.

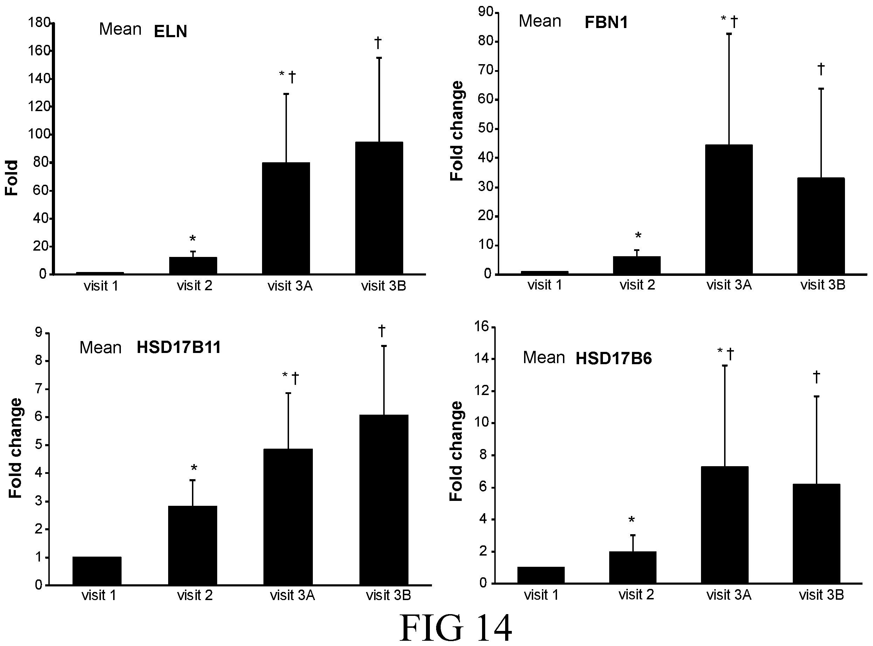

FIG. 14 shows the effect of PrimaVie^{®} Shilajit supplementation on ELN, FBN1, HSD17B11, and HSD17B6 gene expression in skeletal muscles of healthy overweight/Class 1 obese human subjects. mRNA expression levels of ELN, FBN1, HSD17B11, and HSB17B6 in muscle biopsies were measured using RT-PCR. The effect of PrimaVie^{®} Shilajit supplementation (250mg/b.i.d) was measured during the course of the entire visit: 8 weeks of supplementation, then 4 additional weeks of supplementation with exercise (immediately before and after 30-minute final exercise routine). Data are mean ± SEM (n=16); ^{∗}p < 0.005 compared to the baseline visit and †p < 0.05 compared to the 8-weeks visit. No significant differences were observed between the 12-weeks pre- and post-30-minute final exercise periods. Visit 1, baseline visit; Visit 2, after 8 weeks of supplementation; Visit 3A after 12 weeks of supplementation with exercise (before 30 minutes of final exercise); Visit 3B, after 12 weeks of supplementation with exercise (after 30 minutes of final exercise); ELN, elastin; FBN 1, fibrillin 1; HSD17B11, hydroxysteroid (17-Beta) dehydrogenase 11; HSD17B6, hydroxysteroid (17-Beta) dehydrogenase 6.

Thus, the present invention offers a method of using Shilajit, or its individual components, or a combination of two or more of these components to induce the body of a mammal, including the body of a human, to synthesize new collagen thus promoting the health of all the tissues and organs containing collagen, including skin, connective tissue, muscle, cartilage, eye, bone, and teeth, to improve muscle building and regeneration, and/or to treat collagen related disorders.

The product(s) used in the embodiments of the present invention may be formulated into nutraceutical or pharmaceutical dosage forms comprising tablets, capsules, powders, liquids, chews, gummies, transdermals, injectables, etc. using standard excipients and formulation techniques in the industry. The product(s) used in the embodiments of the present invention may be administered to the mammal orally in solid dosage form or by parenteral or transdermal administration.

While in the foregoing specification the present invention has been described in relation to certain embodiments thereof, and many details have been put forth for the purposes of illustration, it will be apparent to those skilled in the art that the invention is susceptible to additional embodiments and that certain of the details described herein can be varied considerably without departing from the basic principles of the invention.

All references cited herein are incorporated by reference in their entireties. The present invention may be embodied in other specific forms without departing from the spirit or essential attributes thereof, and, accordingly, reference should be made to the appended claims, rather than to the foregoing specification, as indicating the scope of the invention.

### STATEMENTS

What is disclosed is:
1. A method of inducing collagen synthesis in a mammal comprising administering to the mammal in need of such treatment a therapeutically effective amount of Shilajit or its individual components, or a combination of two or more of these components, wherein the body of the mammal synthesizes new collagen and/or one or more proteins selected from the group consisting of tenascin, decorin, elastin, myoferlin, fibrillin, and fibronectin.
2. The method of statement 1, wherein the induction of collagen synthesis and the one or more proteins promotes the health of tissues and/or organs containing collagen, wherein the tissues and/or organs are selected from the group consisting of skin, connective tissue, muscle, cartilage, eye, bone, and teeth.
3. The method of statement 1, wherein the mammal is a human, and wherein the induction of collagen synthesis improves muscle building and regeneration.
4. The method of statement 1, wherein the mammal is a human, and wherein the induction of collagen synthesis treats collagen-related disorders selected from the group consisting of osteogenses imperfecta, chondrodysplasias, Ehlers-Danlos Syndrome, Alport Syndrome, osteoporosis, and Knobloch syndrome.
5. The method of statement 1, wherein the individual components of Shilajit comprise 3-hydroxy-dibenzo-α-pyrone, 3,8-dihydroxy-dibenzo-α-pyrone, dibenzo-α-pyrone chromoproteins, humic acid, fulvic acid, and minerals.
6. The method of statement 1, wherein Shilajit has a chemical composition comprising 3-hydroxy-dibenzo-α-pyrone, 3,8-dihydroxy-dibenzo-α-pyrone, dibenzo-α-pyrone chromoproteins, humic acid, fulvic acid, and minerals.
7. The method of statement 1, wherein the mammal is a human, and wherein the dose of Shilajit is from about 20 mg to about 2000 mg per day in humans.
8. The method of statement 7, wherein the mammal is a human, and wherein the dose of Shilajit is from about 100 mg to about 500 mg per day in humans.
9. The method of statement 1, wherein the health of skin, connective tissue, muscle, cartilage, eye, bone, and teeth are improved.
10. The method of statement 1, wherein the wound-healing process is improved.
11. The method of statement 1, wherein the induction of collagen synthesis and/or the one or more proteins is characterized by increased expression of one or more genes selected from the group consisting of: TNXB, DCN, MYOF, COL1A1, COL1A2, COL3A1, MIR3606, COL5A2, COL6A3, COL14A1, ELN, FBN1, and FN1.
12. The method of statement 11, wherein fold change in the expression of the one or more genes is at least about 1.1.
13. The method of statement 11, wherein fold change in the expression of the one or more genes is greater than about 1.5.

## Claims

1. A method of inducing collagen synthesis to improve bone health in a mammal comprising orally administering to the mammal in need of such treatment an amount of Shilajit effective to induce collagen synthesis in the mammal, wherein the body of the mammal synthesizes new collagen and, optionally, one or more proteins selected from the group consisting of tenascin, decorin, elastin, myoferlin, fibrillin, and fibronectin, wherein the tissues and/or organs is bone, and wherein the Shilajit comprises not less than 0.3% dibenzo-α-pyrones, not less than 10.0% dibenzo-α-pyrones conjugated with chromoproteins, and not less than 50% of fulvic acids having a dibenzo-α-pyrone nucleus, less than 1 ppm of lead, less than 1 ppm of arsenic, and less than 0.1 ppm of mercury.

2. The method of claim 1, wherein the mammal is a human, and wherein the dose of Shilajit is from about 20 mg to about 2000 mg per day in humans.

3. The method of claim 2, wherein the mammal is a human, and wherein the dose of Shilajit is from about 100 mg to about 500 mg per day in humans.

4. The method of claim 3, wherein the amount of Shilajit administered is about 250 mg or about 500 mg per day.

5. The method of claim 1, wherein the mammal is a human, and wherein the induction of collagen synthesis treats collagen-related disorders selected from the group consisting of osteogeneses imperfecta, chondrodysplasias, Ehlers-Danlos Syndrome, Alport Syndrome, osteoporosis, and Knobloch syndrome.

6. The method of claim 1, wherein the mammal is a human, and wherein the induction of collagen synthesis treats osteoporosis.
